(19) Europäisches Patentamt
European Patent Office
Office européen des brevets

(11) Veröffentlichungsnummer: **0 078 432**
**A1**

(12) # EUROPÄISCHE PATENTANMELDUNG

(21) Anmeldenummer: **82109577.5**

(22) Anmeldetag: **16.10.82**

(51) Int. Cl.³: **C 07 J 71/00**, A 61 K 31/58

(30) Priorität: **30.10.81 DE 3143757**

(43) Veröffentlichungstag der Anmeldung: **11.05.83**
**Patentblatt 83/19**

(84) Benannte Vertragsstaaten: **AT BE CH DE FR GB IT LI LU NL SE**

(71) Anmelder: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Annen, Klaus, Dr., Seegefelder Strasse 194,**
**D-1000 Berlin 20 (DE)**
Erfinder: **Laurent, Henry, Dr., Glambecker Weg 21,**
**D-1000 Berlin 28 (DE)**
Erfinder: **Hofmeister, Helmut, Dr., Weislingenstrasse 4,**
**D-1000 Berlin 28 (DE)**
Erfinder: **Wendt, Hans, Dr., Ernst-Ring-Strasse 14,**
**D-1000 Berlin 39 (DE)**

(54) **Neue Kortikoide, ihre Herstellung und Verwendung.**

(57) Kortikoide der allgemeinen Formel I

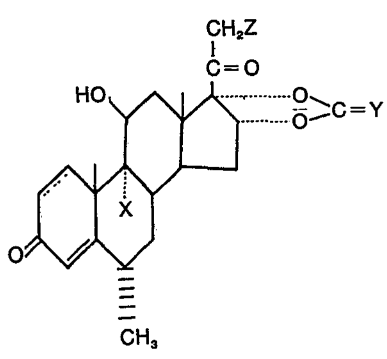

(I),

worin
..... eine Einfachbindung oder eine Doppelbindung,
X ein Wasserstoffatom, ein Fluoratom oder ein Chloratom,
Y zwei Wasserstoffatome oder ein Sauerstoffatom und
Z eine Hydroxygruppe, ein Chloratom oder einen Acyloxyrest mit 2 bis 6 Kohlenstoffatomen darstellen, sind pharmakologisch wirksame Substanzen.

EP 0 078 432 A1

Die Erfindung betrifft den in den Patentansprüchen gekennzeichneten Gegenstand.

Die neuen Kortikoide der allgemeinen Formel I gemäß Patentanspruch 1 können als 2 bis 6 Kohlenstoffatome enthaltende Acyloxygruppen Z beispielsweise eine Acetoxygruppe, eine Propionyloxygruppe, eine Butyryloxygruppe, eine Isobutyryloxygruppe, eine Valeryloxygruppe, eine 3-Methylbutyryloxygruppe, eine Trimethylacetoxygruppe oder eine Hexanoyloxygruppe tragen.

Die im Anspruch 1 gekennzeichneten Kortikoide besitzen bei topischer Applikation eine stark antiinflammatorische Wirksamkeit und sind bei systemischer Applikation relativ schwach wirksam.

Die neuen Kortikoide der allgemeinen Formel I gemäß Patentanspruch 1 eigenen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen Pruritis vulvae et ani, Rosacea, Erythematodes cutaneus, Psoriasis, Lichen ruber planus et verrucosus und ähnlichen Hauterkrankungen.

Die Herstellung der Arzneimnittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die neuen Kortikoide können nach den in dem Patentanspruch 8 gekennzeichneten Verfahren hergestellt werden.

Die nachfolgenden Ausführungsbeispiele dienen zur Erläuterung des erfindungsgemäßen Verfahrens.

Beispiel 1

a) Zu einer Lösung von 80,0 g 21-Acetoxy-16α,17α-dihydroxy-6α-methyl-1,4,9-pregnatrien-3,20-dion in 600 ml wasserfreiem Methylenchlorid und 164 ml wasserfreiem Formaldehyddimethylacetal wird unter Rühren bei Raumtemperatur portionsweise ein Gemisch aus 127 g Kieselgur W 20 und 63,5 g Phosphorpentoxid hinzugefügt. Man rührt 1 Stunde nach, saugt ab, wäscht den Rückstand mit triäthylaminhaltigem Methylenchlorid und engt das Filtrat im Vakuum zur Trockne ein. Man reinigt das Rohprodukt an 8 kg Kieselgel mit einem Methylenchlorid-Aceton-Gradienten (0-12 % Aceton). Ausbeute: 64,3 g 21-Acetoxy-6α-methyl-16α,17α-methylendioxy-1,4,9-pregnatrien-3,20-dion. Schmelzpunkt 176° C.

b) Eine Lösung von 21,0 g 21-Acetoxy-6α-methyl-16α,17α-methylendioxy-1,4,9-pregnatrien-3,20-dion in 210 ml Dioxan wird bei Raumtemperatur unter Rühren mit 19,75 g N-Bromsuccinimid versetzt. Man tropft 105 ml einer 10 %igen wässrigen Perchlorsäure-Lösung hinzu, rührt 10 Minuten bei Raumtemperatur weiter und gibt auf eine Eiswasser-Kochsalz-Lösung. Nach der üblichen Aufarbeitung erhält man 22,4 g 21-Acetoxy-9α-brom-11β-hydroxy-6α-methyl-16α,17α-methylendioxy-1,4-pregnadien-3,20-dion, die aus Aceton/Hexan umkristallisiert werden. Schmelzpunkt 208° C.

c) Eine Suspension von 7,0 g 21-Acetoxy-9α-brom-11β-hydroxy-6α-methyl-16α,17α-methylendioxy-1,4-pregnadien-3,20-dion in 420 ml Äthanol wird mit 9,8 g Kaliumacetat 2 1/2 Stunden refluxiert. Man engt ein und gibt das konzentrierte Reaktionsgemisch auf eine Eiswasser-Kochsalz-Lösung. Nach dem Abfiltrieren und Waschen des Rückstandes arbeitet man wie üblich auf und reinigt das Rohprodukt an 700 g Kieselgel mit einem Methylenchlorid-Aceton-Gradienten (0-15 % Aceton). Ausbeute: 5,7 g 21-Acetoxy-9β,11β-epoxy-6α-methyl-16α,17α-methylendioxy-1,4-pregnadien-3,20-dion. Schmelzpunkt 184° C.

d) 19 ml einer 70 %igen HF/Pyridin-Lösung werden auf -35° C abgekühlt und mit 5,0 g 21-Acetoxy-9β,11β-epoxy-6α-methyl-16α,17α-methylendioxy-1,4-pregnadien-3,20-dion versetzt. Man rührt das Reaktionsgemisch 8 Stunden bei -5° C und gibt anschließend auf ammoniakalisches Eiswasser. Man filtriert ab, wäscht den Rückstand neutral und arbeitet wie üblich auf. Das Rohprodukt wird an 800 g Kieselgel mit einem Methylenchlorid-Aceton-Gradienten (0-15 % Aceton) gereinigt. Es werden 3,7 g 21-Acetoxy-9α-fluor-11β-hydroxy-6α-methyl-16α,17α-methylendioxy-1,4-pregnadien-3,20-dion isoliert. Schmelzpunkt 265° C.

Beispiel 2

2,5 g 21-Acetoxy-9α-fluor-11β-hydroxy-6α-methyl-16α,17α-methylen-dioxy-1,4-pregnadien-3,20-dion werden in einem Gemisch aus 30 ml 60 %iger Perchlorsäure und 100 ml Methanol bei Raumtemperatur 18 Stunden gerührt. Die Reaktionslösung wird mit Methylenchlorid verdünnt und auf Wasser gegeben. Man extrahiert mit Methylenchlorid und erhält nach dem Neutralisieren, Waschen, Trocknen und Einengen der organischen Extrakte ein Rohprodukt, das aus Hexan/Aceton umkristallisiert wird und 1,3 g 9α-Fluor-11β,21-dihydroxy-6α-methyl-16α,17α-methylendioxy-1,4-pregandien-3,20-dion liefert. Schmelzpunkt 231° C.

Beispiel 3

Analog Beispiel 1 b werden 5,0 g 21-Acetoxy-6α-methyl-16α,17α-methylendioxy-1,4,9-pregnatrien-3,20-dion mit N-Chlorsuccinimid umgesetzt, aufgearbeitet und chromatographiert. Ausbeute 3,0 g 21-Acetoxy-9α-chlor-11ß-hydroxy-6α-methyl-16α,17α-methylendioxy-1,4-pregnadien-3,20-dion. Schmelzpunkt 245° C.

Beispiel 4

2,5 g 21-Acetoxy-9α-chlor-11ß-hydroxy-6α-methyl-16α,17α-methylendioxy-1,4-pregnadien-3,20-dion werden analog Beispiel 2 umgesetzt, aufgearbeitet und gereinigt. Man isoliert 920 mg 9α-Chlor-11ß,21-dihydroxy-6α-methyl-16α,17α-methylendioxy-1,4-pregnadien-3,20-dion. Schmelzpunkt 234° C.

Beispiel 5

Eine Lösung von 1,7 g 11ß,21-Dihydroxy-6α-methyl-16α,17α-methylendioxy-1,4-pregnadien-3,20-dion in 16 ml Pyridin und 8 ml Propionsäureanhydrid wird 1 Stunde bei Raumtemperatur gerührt. Nach der Eiswasser-Kochsalz-Fällung wird abfiltriert und wie üblich aufgearbeitet. Das Rohprodukt wird an 150 g Kieselgel mit einem Methylenchlorid-Aceton-Gradienten (0-12 % Aceton) gereinigt. Man erhält 1,6 g 11ß-Hydroxy-6α-methyl-16α,17α-methylendioxy-21-propionyloxy-1,4-pregnadien-3,20-dion. Schmelzpunkt 220° C.

Beispiel 6

Man refluxiert eine Lösung von 15 g 21-Acetoxy-9α-brom-11ß-hydroxy-6α-methyl-16α,17α-methylendioxy-1,4-pregnadien-3,20-dion in 300 ml Tetrahydrofuran mit 103,2 ml Tri-n-butylzinnhydrid und einer Spatelspitze Azobisisobutyronitril eine Stunde lang. Nachdem man die Reaktionslösung im Vakuum zur Trockne eingeengt hat, reinigt man den Rückstand an 1,6 kg

Kieselgel, indem man zunächst mit 7 l Hexan und anschließend mit einem Methylenchlorid-Aceton-Gradienten (0-15 % Aceton) eluiert. Ausbeute 10,0 g 21-Acetoxy-11ß-hydroxy-6α-methyl-16α,17α-methylendioxy-1,4-pregnadien-3,20-dion. Schmelzpunkt 275° C (Zers.).

Beispiel 7

a) Eine Lösung von 1,5 g 9α-Chlor-11ß,21-dihydroxy-6α-methyl-16α,17α-methylendioxy-1,4-pregadien-3,20-dion in 15 ml Pyridin wird 1 Stunde bei Raumtemperatur mit 1,8 g Tosylchlorid gerührt und auf Eiswasser-Kochsalz-Lösung gegeben. Nach der üblichen Aufarbeitung isoliert man 1,8 g 9α-Chlor-11ß-hydroxy-6α-methyl-16α,17α-methylendioxy-21-tosyloxy-1,4-pregnadien-3,20-dion.

b) 1,5 g 9α-Chlor-11ß-hydroxy-6α-methyl-16α,17α-methylendioxy-21-tosyloxy-1,4-pregnadien-3,20-dion werden in 30 ml Hexamethylphosphorsäuretriamid suspendiert und mit 1,5 g Lithiumchlorid 2 1/2 Stunden bei 80° C gerührt. Man gibt auf eine Eiswasser-Kochsalz-Lösung, filtriert ab und arbeitet den Rückstand wie üblich auf. Das Rohprodukt wird an 120 g Kieselgel mit einem Methylenchlorid-Aceton-Gradienten (0-15 % Aceton) gereinigt. Ausbeute: 800 mg 21-Chlor-9α-fluor-11ß-hydroxy-16α,17α-methylendioxy-1,4-pregnadien-3,20-dion. Schmelzpunkt 257° C.

Beispiel 8

Wie im Beispiel 2 beschrieben, werden 9,0 g 21-Acetoxy-11ß-hydroxy-6α-methyl-16α,17α-methylendioxy-1,4-pregnadien-3,20-dion umgesetzt, aufgearbeitet und gereinigt. Ausbeute 5,8 g 11ß,21-Dihydroxy-6α-methyl-16α,17α-methylendioxy-1,4-pregnadien-3,20-dion. Schmelzpunkt 140° C.

Beispiel 9

1,3 g 11ß,21-Dihydroxy-6α-methyl-16α,17α-methylendioxy-1,4-pregnadien-3,20-dion werden analog Beispiel 5 umgesetzt, aufgearbeitet und gereinigt. Man isoliert 1,0 g 11ß-Hydroxy-6α-methyl-16α,17α-methylendioxy-21-propionyloxy-1,4-pregnadien-3,20-dion. Schmelzpunkt 180° C.

Beispiel 10

Unter den Bedingungen des Beispiels 5 werden 1,3 g 11ß,21-Dihydroxy-6α-methyl-16α,17α-methylendioxy-1,4-pregnadien-3,20-dion mit Buttersäureanhydrid umgesetzt, aufgearbeitet und gereinigt. Ausbeute 1,3 g 21-Butyryloxy-11ß-hydroxy-6α-methyl-16α,17α-methylendioxy-1,4-pregnadien-3,20-dion. Schmelzpunkt 170° C.

Beispiel 11

Analog Beispiel 5 werden 1,3 g 11ß,21-Dihydroxy-6α-methyl-16α,17α-methylendioxy-1,4-pregnadien-3,20-dion mit Valeriansäureanhydrid umgesetzt, aufgearbeitet und gereinigt. Man isoliert 1,35 g 11ß-Hydroxy-6α-methyl-16α,17α-methylendioxy-21-valeryloxy-1,4-pregnadien-3.20-dion. Schmelzpunkt 144° C.

Beispiel 12

Analog Beispiel 1 b wird 1,0 g 21-Chlor-6α-methyl-16α,17α-methylendioxy-4,9-pregnadien-3,20-dion mit N-Chlorsuccinimid umgesetzt, aufgearbeitet und chromatographiert. Man isoliert 450 mg 9α,21-Dichlor-11ß-hydroxy-6α-methyl-16α,17α-methylendioxy-4-pregnen-3,20-dion. Schmelzpunkt 225° C.

Patentansprüche

1. Kortikoide der allgemeinen Formel I

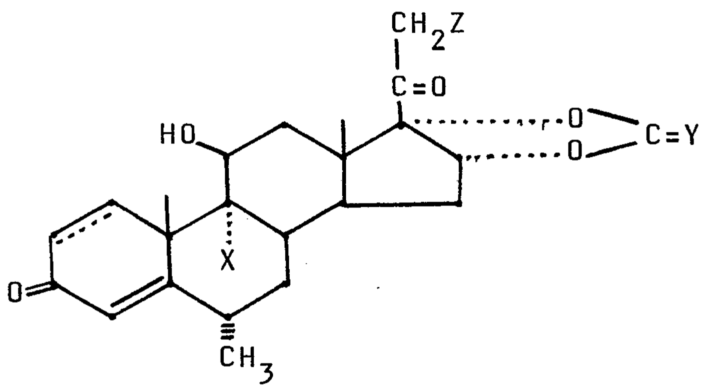

(I),

worin

..... eine Einfachbindung oder eine Doppelbindung,

X ein Wasserstoffatom, ein Fluoratom oder ein Chloratom,

Y zwei Wasserstoffatome oder ein Sauerstoffatom und

Z eine Hydroxygruppe ein Chloratom oder einen Acyloxyrest mit 2 bis 6 Kohlenstoffatomen darstellen.

2. 9α-Fluor-11β,21-dihydroxy-6α-methyl-16α,17α-methylendioxy-1,4-pregnadien-3,20-dion und
   21-Acetoxy-9α-fluor-11β-hydroxy-6α-methyl-16α,17α-methylendioxy-1,4-pregnadien-3,20-dion.

3. 9α-Chlor-11β,21-dihydroxy-6α-methyl-16α,17α-methylendioxy-1,4-pregnadien-3,20-dion,
   21-Acetoxy-9α-chlor-11β-hydroxy-6α-methyl-16α,17α-methylendioxy-1,4-pregnadien-3,20-dion und
   9α-Chlor-11β-hydroxy-6α-methyl-16α,17α-methylendioxy-21-propionyloxy-1,4-pregnadien-3,20-dion.

4. 11β,21-Dihydroxy-6α-methyl-16α,17α-methylendioxy-1,4-pregnadien-3,20-dion,
   21-Acetoxy-11β-hydroxy-6α-methyl-16α,17α-methylendioxy-1,4-pregnadien-3,20-dion,
   11β-Hydroxy-6α-methyl-16α,17α-methylendioxy-21-propionyloxy-1,4-pregnadien-3,20-dion,
   21-Butyryloxy-11β-hydroxy-6α-methyl-16α,17α-methylendioxy-1,4-pregnadien-3,20-dion und
   11β-Hydroxy-6α-methyl-16α,17α-methylendioxy-21-valeryloxy-1,4-pregnadien-3,20-dion.

5. 9α,21-Dichlor-11β-hydroxy-6α-methyl-16α,17α-methylendioxy-1,4-pregnadien-3,20-dion.

6. 9α,21-Dichlor-11β-hydroxy-6α-methyl-16α,17α-methylendioxy-4-pregnen-3,20-dion.

7. Pharmazeutische Präparate gekennzeichnet durch den Gehalt an ein oder zwei Kortikoiden gemäß Anspruch 1 bis 6.

8. Verfahren zur Herstellung von Kortikoiden der allgemeinen Formel I

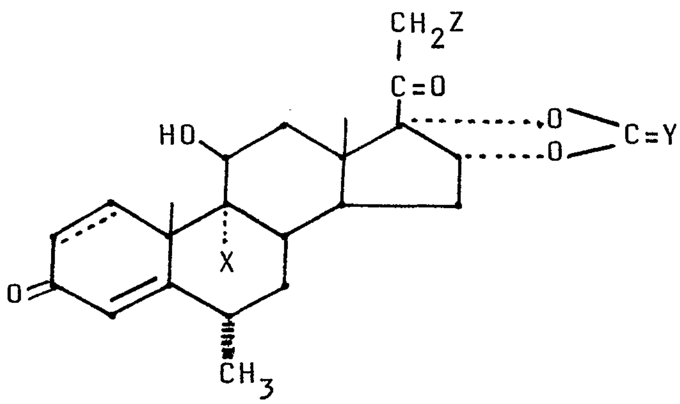

(I),

worin

..... eine Einfachbindung oder eine Doppelbindung,

X ein Wasserstoffatom, ein Fluoratom oder ein Chloratom,

Y zwei Wasserstoffatome oder ein Sauerstoffatom und

Z eine Hydroxygruppe, ein Chloratom oder einen Acyloxyrest mit 2 bis 6 Kohlenstoffatomen darstellen, dadurch gekennzeichnet, daß man

a) ein Kortikoid der allgemeinen Formel II

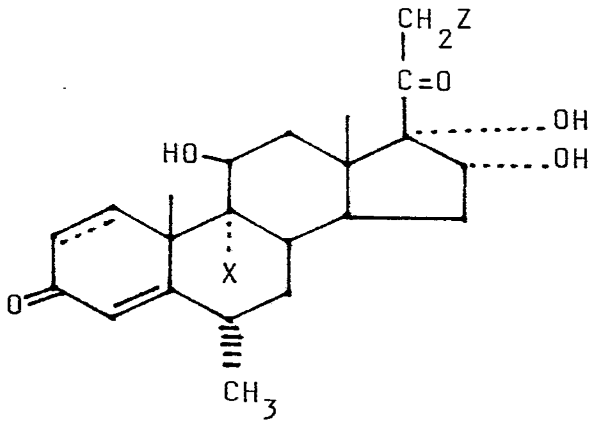

(II),

worin

....., X und Z die obengenannte Bedeutung besitzen,
mit einer Verbindung der allgemeinen Formel III

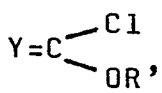

(III),

worin

Y ein Sauerstoffatom und R' jeweils einen 1 bis 4 Kohlenstoffatome enthaltender Alkylrest darstellt, kondensiert, oder

b) an die $\Delta^{9(11)}$-Doppelbindung eines Kortikoids der allgemeinen Formel IV

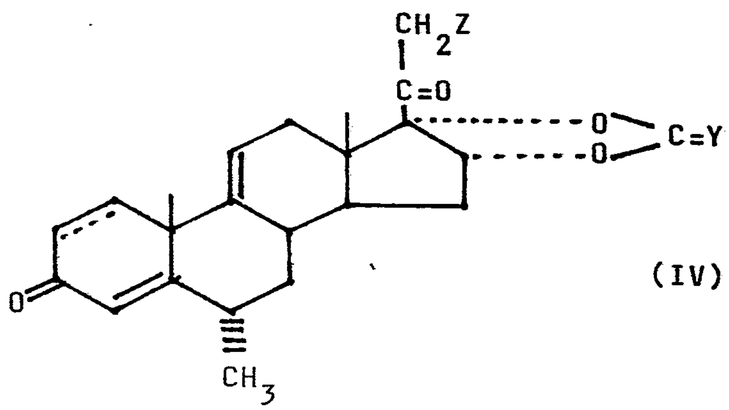

(IV),

worin

....., Y und Z die obengenannte Bedeutung besitzen unterchlorige oder unterbromige Säure anlagert, gegebenenfalls das 9-Halogenatom eliminiert oder die 11ß-Hydroxy-9-halo-

genverbindung in das 9,11ß-Epoxid überführt und dieses mit Fluorwasserstoff oder Chlorwasserstoff öffnet, gewünschtenfalls die gemäß Verfahrensvariante a oder b erhaltenen $\Delta^{1,4}$-Kortikoide der allgemeinen Formel I zu den entsprechenden $\Delta^{4}$-Kortikoiden hydriert, 21-Acetylverbindungen mit Y in der Bedeutung zweier Wasserstoffatome zu den entsprechenden 21-Hydroxyverbindungen verseift und 21-Hydroxysteroide der allgemeinen Formel I verestert oder in die 21-Chlorverbindungen überführt.

Europäisches Patentamt

**EUROPÄISCHER RECHERCHENBERICHT**

0078432

Nummer der Anmeldung

EP 82 10 9577

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-3 069 420 (JOSEF FRIED) * Anspruch 1; Spalten 1-4 * | 1,7,8 | C 07 J 71/00<br>A 61 K 31/58 |
| A | DE-B-1 131 213 (SYNTEX) * Insgesamt * | 1,7,8 | |
| A | US-A-3 050 519 (JOSEF FRIED) * Anspruch 1; Spalten 1-4 * | 1,7,8 | |

RECHERCHIERTE SACHGEBIETE (Int. Cl. 3)

C 07 J 71/00

Der vorliegende Recherchenbericht wurde für alle Patentansprüche erstellt.

| Recherchenort | Abschlußdatum der Recherche | Prüfer |
|---|---|---|
| DEN HAAG | 13-01-1983 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82